# EUROPÄISCHE PATENTANMELDUNG

(11) **EP 1 645 553 A1**
(43) Veröffentlichungstag der Anmeldung: **12.04.2006**
(21) Anmeldenummer: 05020860.2
(22) Anmeldetag: 24.09.2005
(51) Int. Cl.: C07C 67/313, C07C 69/757, C07C 315/04, C07C 205/45, C07F 9/40, C07C 253/30, C07C 45/45, C07C 49/395

(54) **Verfahren zur Herstellung von cyclischen Ketonen**

(30) Priorität: 07.10.2004 DE 102004048875
(71) Anmelder: Bayer MaterialScience AG, 51368 Leverkusen (DE)
(72) Erfinder: Gürtler, Christoph, Dr., 50676 Köln (DE); Kirchhoff, Jörg, Dr., 51061 Köln (DE); Schwarz, Ido, Dr., 40468 Düsseldorf (DE)

(57) **Zusammenfassung**

Die vorliegende Erfindung betrifft ein lösungsmittelfreies Verfahren zur Herstellung cyclischer 1,3-Ketoester, wie z.B. Cyclopentanon-2-carboxyalkylester, durch eine Dieckmann Kondensationsreaktion.

## Beschreibung

Die vorliegenden Erfindung betrifft ein neues Verfahren, um cyclische Ketone wie z.B. Cyclopentanon-2-carboxyalkylester herzustellen. Die Stoffe, die auf diesem Weg gewonnen werden, finden Verwendung als Grundbausteine für Pharmazeutika und Pflanzenschutzmittel, als Feinchemikalien, für die Lackindustrie etc.

Die nach Dieckmann benannte intramolekulare Kondensationsreaktion (Ber.Dtsch. Chem.Ges. 1894(27)965, Liebigs Ann. 1901(317)27) ist bekannt. Beispielsweise führt die Dieckmann-Kondensation von Adipinsäuredialkylestern zu Cyclopentanon-2-carboxyalkylestern. Das Verfahren beruht darauf, dass eine Kondensationsreaktion stattfindet, wobei der jeweilige Alkohol freigesetzt wird:

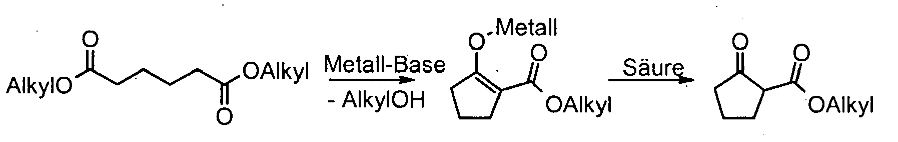

Diese Verfahren zeichnen sich dadurch aus, dass mit fast vollständigem Umsatz und Selektivität das Alkalimetall- bzw. das Erdalkalimetallsalz der gewünschten Verbindung hergestellt wird, das in einem weiterem Schritt sauer aufgearbeitet wird. In der Literatur sind vielfältige Bemühungen diskutiert worden, die Durchführung und die Ausbeuten dieser Reaktion zu optimieren. Diese betreffen sowohl das einzusetzende Kondensationsmittel als auch die verwendeten Verfahrensweisen und Lösungsmittel.

Beispielsweise wurde die Cyclisierung von Adipinsäureestem durch Umsetzung mit stöchiometrischen Mengen einer starken Lewissäure (z.B. AlCl₃, TiCl₂(OTf)₂) und einer Base (z.B. NEt₃) als Kondensationsmittel beschrieben (z.B. Pecanha et al., Quim. Nova 20 (1997) 435; Tanabe et al., Chem. Lett. (1986) 1813). Die Reaktion erfolgt hierbei in Lösung mit guten Ausbeuten, allerdings müssen stöchiometrische Mengen Lewis-Säure und Base (z.B. Triethylamin) verwendet werden. Bei der dann folgenden wässrigen Aufarbeitung wird die eingesetzte Lewis-Säure vollständig hydrolysiert, was unter ökonomischen und ökologischen Gesichtspunkten nicht wünschenswert ist.

Daneben sind vielfach Verfahren beschrieben worden, die mit einer starken Base als Kondensationsmittel die Cyclisierung herbeiführen. Als Basen geeignet sind beispielsweise Alkalimetalle (z.B. Pinkney, Org.Synth. 1937(17)32), Metallhydride (z.B. Bloomfield et. al., Tetrahedron Lett. 1964,2273) oder Metallamide (z.B. Bouveault et. al., Compt. Rend. 146(1908)138).

Als besonders geeignet hat es sich erwiesen, Alkoholate, insbesondere Alkoholate der Alkali- und Erdalkalimetalle, als Basen zu verwenden (z.B. Natriumethylat, Reed et al. J. Chem. Soc. 1954, 2148 oder Magnesiumethylat, Laukkanen, Chem. Ber. 1957 (31) 124). Das Alkoholat wird gewöhnlich als Lösung im korrespondierenden Alkohol in die Reaktion eingebracht. Um eine Umesterung des Reaktionsedukts oder -produkts durch das eingesetzte Alkoholat oder den eingesetzten Alkohol zu vermeiden, werden gewöhnlich zur Dieckmanncyclisierung Materialien mit identischem Substitutionsmuster eingesetzt. (Methylate in Methanol zur Cyclisierung von Methylestem, Ethylate in Ethanol zur Cyclisierung von Ethylestern etc.). Die benötigten Alkoholate werden entweder durch Reaktion des entsprechenden Metalls mit dem Alkohol gewonnen oder durch die Entwässerung von alkoholischer Natron- bzw. Kalilauge gewonnen.

Da Dieckmannreaktionen Gleichgewichtsreaktionen sind, ist es zum Erzielen eines quantitativen Umsatzes notwendig, den während der Reaktion frei werdenden Alkohol ebenso wie den als Lösungsmittel eingesetzten Alkohol quantitativ zu entfernen. Üblicherweise wird eine Dieckmannreaktion daher in unpolaren Lösungsmitteln wie z.B. Toluol oder Xylol durchgeführt und der Alkohol abdestilliert.

Die literaturbekannten Verfahren zur Dieckmanncyclisierung mit Alkoholaten in unpolaren Lösungsmitteln haben für eine wirtschaftliche industrielle Anwendung verschiedene Nachteile. Hinderlich wirkt sich besonders aus, dass im Reaktionsverlauf eine viskose Suspension aus den Edukten der Reaktion, dem Salz des Reaktionsprodukts und dem Lösungsmittel entsteht, die unter technischen Gesichtspunkten nur sehr schlecht gerührt werden kann. Es kann mit dieser Reaktion nur eine niedrige Raum-Zeit-Ausbeute erzielt werden, da zur Erhaltung der Rührbarkeit in sehr großer Verdünnung, die typischerweise nur ca. 10-20 Gew.% der Reaktionspartner und 80-90 Gew.% Lösungsmittel enthält, gearbeitet werden muss.

Aufgrund der hohen Viskosität der Mischung gelingt es darüber hinaus gewöhnlich nicht, in einer vertretbaren Zeit einen vollständigen Umsatz, d.h. 100%ige Reaktion des Adipinsäureesters herbeizuführen. In diesem Fall erhält man nach der Hydrolyse des Reaktionsgemischs ein Produkt, das noch Anteile an Startmaterial aufweist. Dieser kann vom gewünschten cyclischen Ketoncarboxyalkylester destillativ nur sehr schlecht abgetrennt werden, da diese Substanzen ähnliche Siedepunkte aufweisen.

Der Einsatz verschiedener Lösungsmittel wurde beschrieben, welche die Viskosität des Reaktionsgemischs senken und derart die Durchführung der Dieckmanncyclisierung verbessern sollen.

Cassebaum et. al. (DD-A 085560 (1971); Z. Chem. 1971(11)14) beschreiben ein Verfahren, bei dem die Reaktion mit Natriumethylat als Base in einem Lösungsmittelgemisch von o-Dichlorbenzol und Dimethylformamid durchgeführt wird.

Richter Gedeon (HU-A 173512, 1978) beschreiben ebenfalls ein Verfahren, bei dem dipolare, aprotische Lösungsmittel (z.B. Dimethylformamid) für Dieckmannreaktionen eingesetzt werden.

Kao Corp (JP-A 9183755, 1997) führt die Reaktion in Lösungsmittelgemischen von aromatischen organischen Lösungsmittel (z.B. Toluol, Xylol) und in tertiären Alkoholen (z.B. tert.-Butanol, Amylalkohol) durch. Die entsprechenden tertiären Alkoholate als Kondensationsmittel werden durch Umsetzung des tertiären Alkohols mit Natrium oder Natriumhydrid erzeugt.

Verfahren in Gegenwart eines Lösungsmittels haben verschiedene Nachteile. Die Abtrennung und Rückgewinnung des Alkohols aus den eingesetzten Lösungsmitteln ist aufwendig. Anteile von Wasser in den eingesetzten Lösungsmittel reagieren mit den eingesetzten starken Basen und zersetzen diese. Polare Lösungsmittel oder Lösungsmittelgemische sind teilweise teuer und schlecht regenerierbar. Sie sind mit Wasser ganz oder teilweise mischbar, so dass bei der Aufarbeitung des Reaktionsgemischs durch sauere, wässrige Hydrolyse das polare Lösungsmittel oder die polaren Anteile des Lösungsmittelgemisches in der wässrigen Phase gelöst werden. Eine Wiedergewinnung der polaren Lösungsmittel aus der wässrigen Phase ist nur unter großem Aufwand möglich. Darüber hinaus können mit dem polaren Lösungsmittel auch Teile des Reaktionsprodukts in die wässrige Phase verschleppt werden. Bei Verwendung von hochpolaren Lösungsmitteln oder Lösungsmittelgemischen kommt es bei der Hydrolyse zu keiner Phasentrennung, es bildet sich eine einheitliche Wasser/Lösungsmittel/Produkt-Phase, die nur unter sehr großem Aufwand aufgearbeitet werden kann. Die zur Rückgewinnung des Produkts aus der wässrigen Phase notwendige Extraktion mit einem unpolaren Lösungsmittel und die aufwendige Rückgewinnung der hochpolaren Lösungsmittel aus der wässrigen Phase sind wesentliche Nachteile der beschriebenen Verfahrensweise.

Vorteilhaft sind daher Verfahren, die auf den Einsatz eines zusätzlichen Lösungsmittels verzichten. VEB Fahlberg List (DE-A 2055009 (1972)) beschreiben ein Verfahren, in dem die Reaktion ausschließlich im zur Synthese des Kondensationsmittels eingesetzten Alkohol durchgeführt wird. Beispielsweise wird zur Synthese von Cyclopentanon-2-carboxyethylester Magnesiumpulver in einem Überschuss von Ethanol gelöst. Das gebildete Magnesiumethylat wird mit Adipinsäurediethylester versetzt und Ethanol abdestilliert, wobei sich eine harzartige Masse bildet.

Dieses Verfahren hat verschiedene Nachteile. Es ist weiterhin der Einsatz eines Lösungsmittel notwendig. Die für einen quantitativen Umsatz notwendige quantitative Entfernung des Alkohols ist nur unter drastischen Bedingungen möglich, das Gemisch wird bei einer Ansatzgröße von 1,6 mol in 1¾ bis 2½ Stunden auf ca. 220°C erhitzt. Für eine Produktion im großtechnischen Maßstab ist diese Verfahrensweise ungeeignet. Im weiteren Verlauf des offenbarten Verfahrens muss die harzartige Masse vor der Hydrolyse zunächst in Benzol gelöst werden. Damit ist im Verlauf des Verfahrens doch der Einsatz zusätzlicher Lösungsmittel notwendig. Nur 90% des eingesetzten Benzols lassen sich zurückgewinnen.

Besonders vorteilhaft sind daher Verfahren, bei denen auch auf Alkohol zum Lösen des Kondensationsmittels verzichtet werden kann. Toda et al. (J. Chem. Soc., Perkin Trans. 1, 1998/3521) beschreiben eine Methode, bei der auch auf einen Alkohol zum Auflösen des eingesetzten Alkoholats verzichtet wird. Hierbei wird der Adipinsäuredialkylester mit einem festen Alkalimetallalkoholat in einem Mörser mit einem Pistill durchgeknetet. Die Freisetzung des Reaktionsprodukts aus dem Satz erfolgt durch Zugabe von p-Toluolsulfonsäure. Interessanterweise wird bei diesem Beispiel kein vollständiger Umsatz erzielt. Bei der Umsetzung von Adipinsäurediethylester mit Natriumethylat wird eine Ausbeute von 61% angegeben. Eine höhere Ausbeute (82%) wird bei Verwendung von Kalium-tert.-butylat als Kondensationsmittel angegeben.

Für eine Umsetzung im technischen Maßstab hat diese Methode verschiedene Nachteile. Kostengünstige Kondensationsmittel (z.B. Natriumethylat) geben nur mäßige Ausbeuten. Bezogen auf die Größe der Ansätze ist die spezifische mechanische Energie, die über das Pistill eingetragen wird, beträchtlich.

Es bestand somit die Aufgabe, ein technisch einfaches Verfahren zu finden, dass die Herstellung von Cyclopentanon-2-carboxyalkylestern bei geringem ökonomischem Aufwand erlaubt, wobei auf Lösungsmittel gänzlich verzichtet werden sollte, und zugleich eine vollständige Reaktion des Adipinsäurealkylesters erfolgen sollte.

Diese Aufgabe wird durch das erfindungsgemäße Verfahren gelöst. Überraschenderweise wurde gefunden, dass in einem Feststoffreaktor oder in einem Hochviskosreaktor ein vollständiger Umsatz unter Verzicht auf ein Lösungsmittel erzielt werden kann.

Für das erfindungsgemäße Verfahren werden vorteilhaft Feststoffreaktoren, Hochviskosreaktoren oder Mischkneter verwendet, die über eine ausreichende Knetleistung verfügen und eine Vorrichtung zum Abziehen der flüchtigen Reaktionsprodukte besitzen. Vorteilhaft sind zudem solche Maschinen, die evakuiert werden können und / oder zusätzlich beheizbar sind. Die Raum-Zeit Ausbeute wird durch Evakuieren und Beheizen und die damit beschleunigte Entfernung der Flüchtigen deutlich erhöht, was zu einer deutlichen Erhöhung der Wirtschaftlichkeit führt. Die Mischwirkung und der mechanische Eintrag von Energie sind bei diesen Gerätetypen ausreichend, um einen vollständigen Umsatz herbeizuführen.

Das erfindungsgemäße Verfahren kann in ein- oder mehrwelligen Maschinen durchgeführt werden. Zu den einwelligen Maschinen zählen z.B. Mischkneter, Schaufeltrockner oder vergleichbare Apparate, die eine homogene Durchmischung der Edukte und damit einen vollständigen Umsatz gewährleisten. Beispiele für derartige Apparate sind z.B. in EP-A 0 729 780 (Draiswerke), EP-A 0 611 937 (Draiswerke), DE-A 19 747 218 (Lödige), EP-A 0 304 925 (List) beschrieben.

Besonders vorteilhaft wird das erfindungsgemäße Verfahren in mehrwelligen Mischknetern oder Hochviskosreaktoren durchgeführt. Die Wellen besitzen Mischelemente, die ineinandergreifen können, wodurch eine gewisse Selbstreinigung gewährleistet wird. Die Misch- bzw. Knetwellen können gleich- oder gegensinnig rotieren. Durch die Drehbewegung der Wellen und die darauf befestigten Knetelemente wird ein ausreichender Energieeintrag gewährleistet, um eine gute Durchmischung und damit einen vollständigen Reaktionsumsatz zu erzielen. Beispiele für teilweise selbstreinigende Maschinen finden sich z.B. in EP-A 0 329 092 (List) und EP-A 0 517 068 (List). Ein Beispiel für eine vollständig selbstreinigende Maschine wird in EP-A 0 460 466 (Bayer) beschrieben.

Die genannten Maschinen weisen zudem ein großes freies Volumen auf, das für eine effektive Entfernung der flüchtigen Reaktionsprodukte und damit für eine gute Raum-Zeit-Ausbeute notwendig ist. Die genannten Apparate können je nach Konfiguration diskontinuierlich oder kontinuierlich betrieben werden. Für den kontinuierlichen Betrieb sind entsprechende Ein- und Austragsorgane vorzusehen, wie z.B. in WO02/20885 beschrieben.

Das erfindungsgemäße Verfahren kann ebenfalls vorteilhaft in einem mehrwelligen Extruder durchgeführt werden. Mehrwellige Extruder weisen eine extrem gute Mischwirkung und gute Selbstreinigung auf. Gleichläufige oder gegenläufige Mehrwellenextruder sind dem Fachmann bekannt als Maschinen für die Reaktion, die Plasifizierung und Entgasung von pastösen oder hochviskosen Medien oder auch zur Förderung von Feststoffen. Geeignet sind Extruder mit zwei oder mehr Wellen. Auch Planetwalzenextruder sind einsetzbar (z.B. DE-A 10 054 854, Entex). Positiv auf den Prozess wirkt sich ebenfalls die häufige und effektive Oberflächenerneuerung in einem Extruder aus, die einen guten Stoffübergang und damit eine schnelle Entfernung der flüchtigen Bestandteile gewährleistet.

In einem Hochviskosreaktor lassen sich maximale Verweilzeiten von 30min und mehr realisieren, während wirtschaftliche Verweilzeiten in einem Extruder zwischen 10s und 10 min liegen. Die Fahrweise des Prozesses, gegeben durch Temperatur und Druck, muss an die Gegebenheiten der Maschinen angepasst werden. Dem Fachmann ist dabei der Zusammenhang zwischen Verweilzeit, Stoffübergang, Temperatur und Druck bekannt.

Mit dem erfindungsgemäßen Verfahren ist es möglich, beliebige 5-7 Cycloketone herzustellen.

Das erfindungsgemäße Verfahren ist dazu geeignet, CH-azide cyclischen Ketonen der allgemeinen Formel (I), 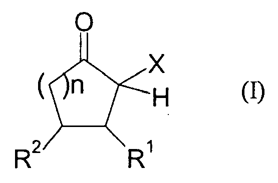 in welcher
- X: eine elektronenziehende Gruppe (Carboxyester oder -Nitril)
- R¹ und R²: unabhängig voneinander die Reste H, C₁-C₂₀-(Cyclo)alkyl, C₆-C₂₄-Aryl, C₁-C₂₀- (Cyclo)alkylester oder -amid, C₆-C₂₄-Arylester oder -amid, gemischt aliphatisch/aromatische Reste mit 1 bis 24 Kohlenstoffatomen, die auch Teil eines 4 bis 8-gliedrigen Ringes sein können, darstellen,
- n: eine ganze Zahl von 0 bis 3 ist,
bei vollständigem Umsatz der zugrundeliegenden Esterkomponente zu erzeugen.

Bei der elektronenziehenden Gruppe X kann es sich um alle Substituenten handeln, die zu einer CH-Azidität des α-ständigen Wasserstoffes führen. Dies können beispielsweise Estergruppen, Nitrilgruppen oder Carbonylgruppen sein. Bevorzugt sind Estergruppen, besonders bevorzugt der Carbonsäuremethylester- und Carbonsäureethylestergruppen.

Geeignet sind auch Verbindungen der allgemeinen Formel (I), deren Ring gegebenenfalls Heteroatome, wie Sauerstoff-, Schwefel-, oder Stickstoffatome enthalten.

Bevorzugt weist das aktivierte cyclische Keton der Formel (I) eine Ringgröße von 5 (n = 1) und 6 (n = 2) auf.

Bevorzugte Verbindungen der allgemeinen Formel (I) sind Cyclopentanon-2-carboxymethylester und -carboxyethylester, Cyclopentanon-2-carbonsäurenitril, Cyclohexanon-2-carboxymethylester und -carboxyethylester oder Cyclopentanon-2-carbonylmethyl. Besonders bevorzugt sind Cyclopentanon-2-carboxymethylester und -carboxyethylester sowie Cyclohexanon-2-carboxymethylester und -carboxyethyl-ester.

Die Cyclopentanonsysteme sind technisch leicht durch die oben aufgeführte Dieckmann Kondensation von Adipinsäuredimethylester oder Adipinsäurediethylester erhältlich.

Die Erfindung wird durch folgende Beispiele beschrieben.

### Beispiele

### Herstellung cyclischer 1,3-Diketoverbindungen

### Beispiel 1

Das Verfahren wurde in einem Hochviskosreaktor der Fa. List AG vom Typ CRP 2,5 Batch durchgeführt. Der Hochviskosreaktor ist eine Maschine mit zwei horizontal angeordneten gleichsinnig drehenden Mischwellen. Auf den Wellen befinden sich Knetorgane, die ineinander greifen und damit eine schnelle und homogene Vermischung gewährleisten. Zusätzlich besitzt die Maschine eine Austragsschnecke, über die das Produkt aus dem Reaktionsraum gefördert werden kann. Ein Betrieb ist kontinuierlich und diskontinuierlich möglich. Der Reaktor hat ein freies Volumen von ca. 2,51. Der Reaktionsraum der Maschine ist beheizbar. Über einen Entgasungsdom können flüchtige Bestandteile abgezogen werden. In den Hochviskosreaktor wurden 1336g Adipinsäurediethylester und 472g Natrium-ethylat vorgelegt. Nach dem Start des Kneters und der Durchmischung der Edukte bildete sich unmittelbar eine zähe Masse. Unter langsamem Kneten wurde langsam auf eine Temperatur von 120°C erhitzt. Dabei wurde langsam ein Vakuum von 10 mbar aufgebaut. Die Temperatur von 120°C wurde nach ca. 15 Minuten erreicht. Man ließ danach noch bei einer Temperatur von 120°C 30 Minuten langsam weiterkneten, bis ein pulversierter weißer Feststoff vorlag. Das entstandene Pulver wurde über eine Förderschnecke ausgetragen und anschließend mit halbverdünnter Schwefelsäure hydrolysiert. Nach Phasentrennung und erfolgter Destillation bei 120°C/10 mbar erhielt man 1021 g Cyclopentanon-2-carboxyethylester, ca. 99% der Theorie. Es konnte kein Adipinsäurediethylester mehr nachgewiesen werden.

### Beispiel 2

Das Verfahren wurde in einem gleichsinnig drehenden Doppelwellenextruder vom Typ ZSK34 mit einem Wellendurchmesser von 34mm durchgeführt. Der Verfahrensteil der Maschine hatte eine Länge von 1560mm. Die Maschine war mit einer Öffnung für die Feststoffzufuhr ausgestattet. Die Flüssigkeit wurde über eine Gehäusebohrung zudosiert. Die Vermischung der Komponenten erfolgt durch die sich auf der Welle befindenen Knetelemente. Die flüchtigen Reaktionsprodukte wurden über ein offenes Entgasungsgehäuse abgezogen. In die Maschine wurden 1,31 kg/h Natriumethylat als Pulver und 3,69 kg/h Adipinsäurediethylester als Flüssigkeit eindosiert. Die Drehzahl der Maschine betrug ca. 60 1/min. Durch die Gehäuseheizung wurde das Gemisch auf ca. 150°C erwärmt. Am Ende der Maschine wurde ein krümeliger weißer Feststoff ausgetragen. Nach ansäuern, wässriger Aufarbeitung und Extraktion mit einem organischen Lösungsmittel (Toluol) war kein Edukt in Form von Adipinsäurediethylester, Adipinsäuremonomethylester oder Adipinsäure festzustellen. Lediglich Cyclopentanon-2-carboxyethylester wurde gefunden. Die Ausbeute bezogen auf Adpinsäurediethylester war quantitativ.

## Patentansprüche

1. Verfahren zur Herstellung cyclischer Ketone der allgemeinen Formel (I), 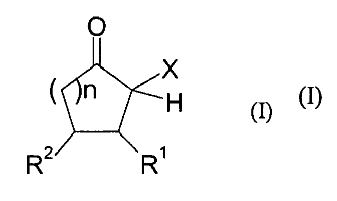 in welcher
X eine elektronenziehende Gruppe
R¹ und R² unabhängig voneinander die Reste H, C₁-C₂₀-(Cyclo)alkyl, C₆-C₂₄-Aryl, C₁-C₂₀-(Cyclo)alkylester oder -amid, C₆-C₂₄-Arylester oder -amid, gemischt aliphatisch/aromatische Reste mit 1 bis 24 Kohlenstoffatomen, die auch Teil eines 4 bis 6-gliedrigen Ringes sein können,
**dadurch gekennzeichnet, dass** eine Metallbase mit einer Verbindung der allgemeinen Formel (II) 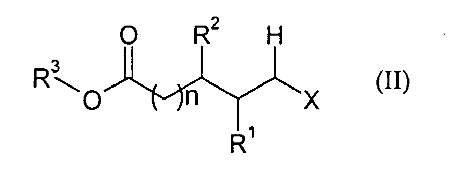 in der unabhängig voneinander die Reste
R¹ und R² die oben angegebene Bedeutung haben,
R³ einen Alkyl- oder Arylrest sowie
X eine elektronenziehenden Gruppe darstellt
in einem Feststoff- oder Hochviskosreaktor umgesetzt wird, ohne dass zusätzliche Lösungsmittel Verwendung finden.

2. Verfahren gemäß Anspruch 1, **dadurch gekennzeichnet, dass** die elektronenziehenden Gruppe X in den allgemeinen Formeln (I) und (II) ausgewählt ist aus der Gruppe der Ester-Sulfoxid-, Sulfon-, Nitro-, Phosphonat-, Nitril-, Isonitril- oder Carbonylgruppen.

3. Verfahren gemäß Anspruch 1, **dadurch gekennzeichnet, dass** die elektronenziehenden Gruppe X in den allgemeinen Formeln (I) und (II) ausgewählt ist aus der Gruppe der Ester- und Nitrile

4. Verfahren gemäß Anspruch 1, **dadurch gekennzeichnet, dass** als Edukte Adipinsäurediethylester oder Adpinsäuredimethylester eingesetzt werden.

5. Verfahren gemäß Anspruch 4, **dadurch gekennzeichnet, dass** als Basen Metallalkoholate eingesetzt werden.

6. Verfahren gemäß Anspruch 1, **dadurch gekennzeichnet, dass** als Feststoffreaktor ein Schaufeltrockner, ein ein- oder mehrwelliger Hochviskosreaktor oder ein ein- oder mehrwelliger Extruder verwendet wird.

7. Verfahren nach einem der vorhergehenden Ansprüche, **dadurch gekennzeichnet, dass** die Reaktion und anschließende Entfernung der flüchtigen Reaktionsprodukte bei einem Druck kleiner oder gleich Atmosphärendruck und / oder bei einer Temperatur größer als 20°C durchgeführt wird.
